# FASCICULE DE BREVET EUROPEEN

(11) **EP 1 143 920 B1**
(45) Date de publication et mention de la délivrance du brevet: **27.08.2003**
(21) Numéro de dépôt: 99959480.7
(22) Date de dépôt: 14.12.1999
(51) Int. Cl.: A61K 7/00, A61K 7/48, A61K 9/107

(54) **EMULSION HUILE-DANS-EAU COMPRENANT UN AGENT BIOLOGIQUEMENT ACTIF MICRONISE ET UN SYSTEME EMULSIONNANT APPROPRIE**
ÖL-IN-WASSER EMULSION DIE EINEN MIKRONISIERTEN BIOLOGISCH-AKTIVEN WIRKSTOFF UND EIN EMULGIERMITTEL ENTHÄLT
OIL-IN-WATER EMULSION COMPRISING A MICRONISED BIOLOGICALLY ACTIVE AGENT AND AN APPROPRIATE EMULSIFIER SYSTEM

(30) Priorité: 18.12.1998 FR 9816050
(43) Date de publication de la demande: 17.10.2001
(73) Titulaire: Galderma Research & Development, S.N.C., 06560 Valbonne (FR)
(72) Inventeur: SEGURA, Sandrine, F-06410 Biot (FR); PREUILH, Isabelle, F-06110 Le Cannet (FR)
(74) Mandataire: Renard, Emmanuelle
(86) Numéro de dépôt international: FR9903136
(87) Numéro de publication internationale: WO00037027

(56) Documents cités:
- US-A- 5 073 372
- US-A- 5 223 559
- US-A- 5 306 485
- US-A- 5 612 021
- US-A- 5 833 999

## Description

La présente invention concerne une composition cosmétique ou pharmaceutique se présentant sous la forme d'une émulsion huile-dans-eau comprenant un agent biologiquement actif micronisé et un système émulsionnant approprié destinée, en application topique, au traitement ou au soin de la peau et/ou de ses phanères.
En particulier la composition de l'invention permet de favoriser la pénétration de l'agent biologiquement actif à la base du follicule pileux.
La présente invention concerne également une composition pour traiter et/ou prévenir toutes les affections liées à une inflammation ou une infection des tissus environnants le follicule pileux.
Dans l'art antérieur, dans le traitement de l'acné par exemple, on connaît de nombreuses compositions dermatologiques comprenant un agent actif. Pour diverses raisons liées en particulier à l'excès de sébum et aux tendances grasses des peaux acnéiques, ces compositions se présentent le plus souvent sous la forme de gels aqueux. Tout en présentant un toucher non gras et une sensation de fraîcheur, les gels aqueux présentent l'inconvénient de procurer, à grande fréquence d'utilisation, une sensation de tiraillement de la peau ce qui est également inconfortable.
Un des buts de l'invention est de procurer une composition topique qui ne présente pas ces inconvénients et qui procure des qualités de confort à l'application et tout au long du traitement.
Dans les compositions cosmétiques ou pharmaceutiques, le ou les principes actifs sont généralement sous forme solubilisée. Or il se trouve que certains principes actifs sont peu solubles à un pH compris entre 5 et 7, c'est à dire à un pH compatible avec la peau et à un pH idéal pour une composition hautement tolérée. Leur utilisation sous forme solubilisée à un tel pH sans incorporation d'additif n'est donc pas possible. Il faut donc que le principe actif soit dans un état thermodynamique différent de la solubilisation.

De plus, pour des raisons d'efficacité ou pour éviter l'apparition d'effets secondaires indésirables, il est parfois préférable de faire accéder le principe actif sélectivement dans des zones-cibles.

Ceci peut-être le cas par exemple pour le traitement de certaines affections de la peau et/ou de ses phanères où il est préférable de faire accéder le principe actif sélectivement à la base des follicules pileux, telles que pour le traitement des affections dermatologiques liées à une inflammation ou d'une infection des tissus environnants le follicule pileux. Parmi ces affections, on peut citer notamment l'acné et la folliculite.

C'est un autre but de l'invention que de procurer un moyen de favoriser l'accès du principe actif là où il doit agir, sans modifier son activité et sa compatibilité cutanée.

La demanderesse a découvert, de manière inattendue, que l'utilisation d'un composé biologiquement actif sous forme micronisé et non solubilisé, en dispersion dans une émulsion de type huile-dans-eau comprenant un système émulsionnant approprié permet d'obtenir une composition pharmaceutique ou cosmétique ne présentant pas les inconvénients des compositions de l'art antérieur.

La présente invention a donc pour objet une composition cosmétique ou pharmaceutique du type émulsion huile-dans-eau comprenant une phase grasse dispersée dans une phase aqueuse, caractérisée par le fait qu'elle comprend :
A) au moins un composé biologiquement actif micronisé, non solubilisé, sous forme de particules, dont au moins 80% en nombre de particules et de préférence au moins 90% en nombre de particules ont un diamètre compris entre 1 à 10 µm et au moins 50 % en nombre de particules ont un diamètre inférieur à 5 µm, et
B) un système émulsionnant approprié,

Les émulsions selon l'invention présentent l'avantage d'être compatibles avec la peau, confortables à l'application, sans présenter un caractère gras ou collant, tout en favorisant la pénétration sélective du composé biologiquement actif dans les follicules pileux, ce qui augmente son efficacité tout en diminuant les effets secondaires indésirables.

Un autre avantage que présente les émulsions de l'invention est qu'il n'est pas nécessaire d'encapsuler le composé biologiquement actif, technique utilisée dans l'art antérieur pour obtenir un ciblage folliculaire. L'absence d'encapsulation permet de simplifier le procédé de fabrication du composé actif et de réduire ainsi les coûts.
Selon l'invention, d'une manière avantageuse, dans la composition, le système émulsionnant comprend au moins un copolymère constitué d'une fraction majoritaire de monomère d'acide carboxylique monooléfiniquement insaturé en C₃-C₆ ou de son anhydride et d'une fraction minoritaire de monomère ester à chaîne grasse d'acide acrylique.
Les copolymères émulsionnants conformes à la présente invention sont préparés en polymérisant une quantité prépondérante de monomère d'acide carboxylique monooléfiniquement insaturé ou de son anhydride, à une quantité plus faible de monomère ester à chaîne grasse d'acide acrylique. La quantité de monomère d'acide carboxylique ou de son anhydride est de préférence comprise entre 80 et 98% en poids et plus particulièrement entre 90 et 98% en poids tandis que l'ester à chaîne grasse d'acide acrylique est présent dans des quantités comprises entre 2 et 20% en poids et plus particulièrement entre 1 et 10% en poids ; les pourcentages étant calculés par rapport au poids des deux monomères.

Les monomères d'acide carboxylique préférentiel sont choisis parmi ceux répondant à la formule suivante : 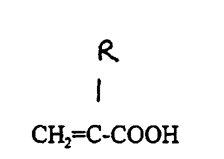 où R désigne hydrogène, halogène, hydroxyle, un groupe lactone, un groupe lactame, un groupe cyanogène (-C=N), un groupe alkyle monovalent, un groupe aryle, un groupe alkylaryle, un groupe aralkyle ou un groupe cycloaliphatique.

Les monomères d'acide carboxylique particulièrement préférés sont choisis parmi l'acide acrylique, l'acide méthacrylique ou leurs mélanges.

Les monomères esters à chaîne grasse d'acide acrylique sont préférentiellement choisis parmi ceux répondant à la formule : 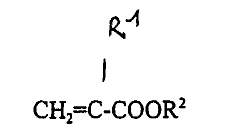 où R¹ est choisi dans le groupe formé par hydrogène, méthyle et éthyle et R² est un groupe alkyle en C₈-C₃₀.
Les monomères esters particulièrement préférés sont ceux pour lesquels R' est hydrogène ou méthyle et R² est un groupe alkyle en C₁₀-C₂₂.

Les copolymères émulsionnants de l'invention sont décrits dans la demande de brevet EP-A-0268164 et sont obtenus selon les méthodes de préparation décrites dans ce même document.

On utilise plus particulièrement le copolymère acrylate/C₁₀-C₃₀-alkylacrylate tel que le produit vendu sous le nom PEMULEN TR 1 ou le produit vendu sous le nom CARBOPOL 1342 par la Société GOODRICH ou bien leurs mélanges.

Les émulsions de l'invention peuvent contenir également d'autres émulsionnants tensioactifs. Parmi ces composés, on peut citer à titre d'exemples le glyceryl(and) PEG100 stéarate vendu sous le nom de Arlacel 165 par la société ICI ou sous le nom de Simulsol 165 par la société SEPPIC, des esters d'acide gras polyoxyéthylénés tel que l'Arlatone 983 de la société ICI, ou l'alcool stéarylique polyoxyéthyléné (2) vendu sous le nom de Brij72 associé à l'alcool stéarylique polyéthyléné (21) vendu sous le nom de Brij721 par la société ICI.

Les émulsions de l'invention peuvent contenir en plus des cotensioactifs. Parmi ces composés, on peut citer à titre d'exemples les esters de sorbitan tels que l'oléate de sorbitan vendu sous le nom de Arlacel 80 par la société ICI ou vendu sous le nom de Crill 4 par la société Croda, le sesquioleate de sorbitan vendu sous le nom de Arlacel 83 par la société ICI ou vendu sous le nom de Montane 83 par la société SEPPIC, ou bien l'isostéarate de sorbitan; les éthers d'alcools gras ayant un haut HLB, c'est à dire un HLB supérieur ou égal à 7 tels que le ceteareth-20 ou le ceteareth 12, ou les éthers d'alcools gras ayant un HLB bas, c'est à dire un HLB inférieur à 7, tel que le steareth-2.

La composition selon l'invention comprend avantageusement jusqu'à 15% en poids de système émulsionnant approprié, de préférence de 0,05 à 8% en poids et plus particulièrement de 0,1 à 2 % en poids par rapport au poids total de la composition.

Dans le système émulsionnant, la quantité de copolymère peut être par exemple de 0,01 à 3 % en poids par rapport au poids total de la composition. Lorsque le système émulsionnant est un copolymère acrylate/C₁₀-C₃₀-alkylacrylate, la quantité de copolymère est de préférence de 0,05 à 2%, et plus particulièrement de 0,1 à 0,5% en poids par rapport au poids total de la composition.

On peut plus particulièrement utiliser comme actif tout actif insoluble ou difficilement soluble dans l'eau ou un milieu hydrophile dans des conditions de pH compatible avec la peau, c'est à dire un pH compris entre 5 et 7, et susceptible d'être micronisé.

Par composé biologiquement actif on entend un composé susceptible de modifier ou moduler le fonctionnement d'au moins un système biologique donné.

Parmi les agents biologiquement actifs, on peut citer à titre d'exemple les agents modulant la différenciation et/ou la prolifération et/ou la pigmentation cutanée, les antibactériens, les antiparasitaires, les antifongiques, les antibiotiques, les agents anti-inflammatoires stéroïdiens, les agents anti-inflammatoires non-stéroïdiens, les agents anesthésiques, les agents antiprurigineux, les agents antiviraux, les agents kératolytiques, les agents anti-radicaux libres, les antiséborrhéiques, les antipelliculaires, les antiacnéiques, les antimétabolites, les agents pour lutter contre la chute des cheveux et pour favoriser la repousse ou l'inverse, les antiseptiques ou leurs mélanges.

Parmi les agents modulant la différenciation et/ou la prolifération, on peut citer par exemple les rétinoides. Parmi les rétinoides, on peut citer d'une manière générale sans que cette liste soit limitative l'adapalène, l'acide rétinoique tout trans, les rétinoides acides ayant donc au moins une fonction carboxylique. Parmi les rétinoides acides, on peut citer par exemple l'acide 6-[7-(1-adamantyl)-6-methoxyethoxymethoxy-2-naphtyl] nicotinique, l'acide 6-[3-(1-adamantyl-4-hydroxyphenyl]-2-naphtoique, l'acide 6-(3,5,5,8,8-pentamethyl-5,6,7,8-tetrahydro-2-naphtylthio)nicotinique, l'acide 3-(3,5,5,8,8-pentamethyl-5,6,7,8-tetrahydro-2-naphtyl)phenylacrylique, l'acide 2-hydroxy-4-[7-(1-adamantyl)-6-methoxyethoxymethoxy-2-naphtyl] benzoique, ou leurs mélanges.

Parmi les antibiotiques, on peut citer par exemple les fluoroquinolones, la rifamycine, la josamycine, la sulfadiazine, la virginiamycine, l'acide fusidique ou leurs mélanges. On préfère utiliser les fluoroquinolones et plus particulièrement la nadifloxacine.

Parmi les antibactériens on peut citer par exemple le peroxyde de benzoyle.

Parmi les antipelliculaires on peut citer par exemple la piroctone olamine.

Parmi les agents kératolytiques on peut citer à titre d'exemple l'acide salicylique.

Parmi les antiradicaux libres on peut citer à titre d'exemple la vitamine E.

Parmi les antiparasitaires, on peut citer par exemple le crotamiton.

Parmi les antiviraux, on peut citer par exemple la Vidarabine.

Parmi les antifongiques, on peut citer par exemple la griseofulvine, les composés appartenant à la classe des imidazoles tels que l'éconazole, le kétoconazole ou le miconazole, les composés polyènes, tels que l'amphotéricine B, les composés de la famille des allylamines, tels que la terbinafine, ou encore le Piroctone olamine.

Parmi les agents anti-inflammatoires stéroïdiens, on peut citer par exemple le Clobetasone butyrate, l'hydrocortisone, la fluocinolone acetonide, la betamethasone.

Les compositions de l'invention conviennent particulièrement bien dans les domaines de traitement suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation telles que la rosacée,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique,
7) pour prévenir ou guérir les stigmates de l'atrophie épidermique et/ou dermique induite par les corticostéroïdes locaux ou systémiques, ou toute autre forme d'atrophie cutanée,
8) pour traiter de manière préventive ou curative les troubles de la cicatrisation, pour prévenir ou pour réparer les vergetures, ou encore pour favoriser la cicatrisation,
9) pour lutter contre les troubles de la fonction sébacée tels que l'hyperséborrhée de l'acné ou la séborrhée simple,
10) dans le traitement préventif ou curatif des états cancéreux ou précancéreux,
11) dans le traitement d'affections inflammatoires telles que l'arthrite,
12) dans le traitement de toute affection d'origine virale au niveau cutané,
13) dans le traitement préventif ou curatif de l'alopécie,
14) dans le traitement d'affections dermatologiques à composante immunologique,
15) dans le traitement de désordres cutanés dus à une exposition aux rayonnements U.V.,
16) dans le traitement d'affections dermatologiques liées à une inflammation ou une infection des tissus environnants le follicule pileux, notamment dues à une colonisation ou infection microbienne notamment l'impétigo, la dermite seborrhéique, la folliculite, le sycosis barbae ou impliquant tout autre agent bactérien ou fongique,
17) dans les traitements cosmétiques destinés à accélérer ou favoriser la chute du poil.

Les compositions de l'invention sont particulièrement adaptées au traitement, de manière préventive ou curative, de l'acné.

Pour le traitement de l'acné, les composé biologiquement actifs sont choisis, de préférence, dans le groupe formé par les antibiotiques, les antibactériens, les antifongiques, les antiparasitaires, les rétinoides, ou leurs mélanges.

Bien entendu, la quantité de composé biologiquement actif dans la composition selon l'invention dépendra du composé biologiquementactif considéré et de la qualité du traitement désiré.

Pour donner un ordre de grandeur, la composition selon l'invention comprend entre 0,0001 et 20 % en poids par rapport au poids total de la composition d'un composé biologiquement actif, de préférence entre 0,025 et 15 % en poids.

Par exemple, dans les compositions pour le traitement de l'acné, les composés biologiquement actifs sont utilisés, de préférence dans les émulsions de l'invention, à des concentrations allant de 0,1 à 10 % en poids et plus particulièrement de 0,5 à 2 % en poids par rapport au poids total de la composition.

Le composé biologiquement actif micronisé peut être obtenu par différentes méthodes comme par exemple la méthode à jet d'air.

La distribution de la taille des particules du composé biologiquement actif est telle que au moins 80% en nombre et de préférence au moins 90% en nombre des particules ont un diamètre compris entre 1 à 10 µm et au moins 50 % en nombre des particules ont un diamètre inférieur à 5 µm. Le diamètre moyen des particules du composé biologiquement actif ainsi micronisé est avantageusement compris entre 3 et 5 µm. De préférence, au moins 30% en nombre des particules ont un diamètre compris entre 3 et 5 µm, et de manière encore plus préférentielle au moins 50% en nombre des particules ont un diamètre compris entre 3 et 5µm.

Le composé biologiquement actif micronisé est non solubilisé dans la composition de l'invention. On entend par non solubilisé, un composé biologiquement actif qui est dissous à moins de 0,05% et de préférence à moins de 0, 01% en poids par rapport au poids de chacun des autres composés pris individuellement de la composition.

La phase grasse de l'émulsion selon l'invention peut comprendre des corps gras usuellement utilisés dans le domaine d'application envisagé. Ils sont choisis de telle manière qu'ils ne soient pas solubilisants de l'agent biologiquement actif au pH compatible avec la peau.

Parmi les corps gras, on peut citer les corps gras siliconés tels que les huiles de silicone, ainsi que les corps gras non siliconés tels que les huiles végétales, minérales, animales ou synthétiques.

Parmi les corps gras siliconés, on peut citer les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA); les huiles siliconées volatiles, telles que les silicones volatiles cycliques ayant de 3 à 8 atomes de silicium et de préférence de 4 à 5, comme par exemple une cyclomethicone telle que la cyclotétradiméthylsiloxane, la cyclopentadiméthylsiloxane ou la cyclohexadiméthylsiloxane, les cyclocopolymères du type diméthylsiloxane/méthylalkylsiloxane, les silicones volatiles linéaires ayant de 2 à 9 atomes de silicium, comme par exemple de l'hexaméthyldisiloxane, de l'hexyl heptaméthyltrisiloxane ou de l'octyl heptaméthyltrisiloxane; les huiles de silicone phénylées.

Parmi les corps gras non siliconés, on peut citer les huiles usuelles, telles que l'isohexadécane, l'huile de paraffine, de vaseline, le perhydrosqualène, l'huile d'abricot, l'huile de germes de blé, d'amande douce, de calophyllum, de palme, de ricin, d'avocat, de jojoba, d'olive ou de germes de céréales; des esters d'acides gras ou d'alcools gras, telles que la diisopropyladipate, l'octyl dodécyl myristate ou les benzoates d'alkyle en C₁₂-C₁₅,; des acétylglycérides; des octanoates, décanoates ou ricinoléates d'alcools ou de polyalcools; des triglycérides d'acides gras; des glycérides; le polyisobutène hydrogéné, des huiles hydrogénées concrètes à 25°C; des lanolines; des esters gras concrets à 25°C. Comme autres corps gras, on peut citer les acides gras tel que l'acide stéarique, les alcools gras tel que l'alcool stéarylique ou cétylique ou leurs dérivés, les cires ou leurs mélanges.

Ces corps gras peuvent en particulier être choisis de manière variée par l'homme du métier afin de préparer une composition ayant les propriétés souhaitées, par exemple en consistance ou en texture.

Ainsi, la phase grasse de l'émulsion selon l'invention peut être présente à une teneur comprise entre 5 et 50 % en poids par rapport au poids total de la composition et de préférence comprise entre 12 et 25 % en poids.

Lorsqu'il s'agit d'une composition destinée au traitement de l'acné, les corps gras sont préférentiellement choisis parmi les huiles sèches à moyennement sèches, à des teneurs préférentielles allant de 5 à 30 % en poids et plus particulièrement de 12 à 25 % en poids par rapport au poids total de la composition.

On entend par huile sèche à moyennement sèche, une huile qui ne donne pas de sensation de gras sur la peau et/ou qui ne laisse pas un film gras sur la peau.

Les huiles sèches à moyennement sèches sont choisies par exemple parmi l'isohexadecane vendu sous le nom d'Arlamol HD par la société ICI, le dioctyl cyclohexane vendu sous le nom de Cetiol S par la société HENKEL, l'isopropyl palmitate vendu sous le nom de Crodamol IPP par la société CRODA, le polyisobutène hydrogéné vendu sous le nom de Polysynlane par la société NOF, le diisopropyladipate vendu sous le nom de Ceraphyl 230 par la société ISP Van Dyk, le dicaprylyl ether vendu sous le nom de Cetiol OE par la société HENKEL, l'isopropyl myristate vendu sous le nom de Crodamol IPM par la société CRODA, le dipropylène glycol diperlargonate vendu sous le nom de DPPG par la société GATTEFOSSE, l'alkyl benzoate en C₁₂₋₁₅ vendu sous le nom de Finsolv TN par la société FINETEX, le cetostearyl isononanoate vendu sous le nom de Cetiol SN par la société HENKEL, le cetostearyl ethylhexanoate vendu sous le nom de Crodamol CAP par la société CRODA, le squalene synthetique vendu sous le nom de Isolan RS par la société GOLDSCHMIDT, l'huile d'olive, l'octyl palmitate vendu sous le nom de Crodamol OP par la société CRODA, l'octyl dodecyl myristate vendu sous le nom de MODWL2949 par la société GATTEFOSSE, le caprylic / capric triglycérides vendu sous le nom de Miglyol 812 par la société HULS ou vendu sous le nom de Myritol 318 par la société HENKEL.

D'autres huiles sèches à moyennement sèches peuvent être utilisées de telle sorte qu'elles présentent des caractéristiques sensorielles équivalentes à celles citées ci-dessus.

Ainsi, à titre d'exemple, d'autres huiles sèches à moyennement sèches pouvant être utilisées dans les émulsions selon l'invention sont par exemple choisies dans le groupe formé par des esters tels que l'isopropyl palmitate, des diesters tels que le diisopropyl adipate vendu par la société ISP Van Dyk sous le nom de Ceraphyl 230, ou vendu par la société Croda sous le nom de Crodamol DA, des éthers tels que le dicaprylyl ether et des polyéthers, des hydrocarbures tel que le polyisobutène hydrogéné vendu sous le nom de polysynlane par la société NOF ou l'isohexadécane vendue par la société ICI sous le nom de Arlamol HD, des huiles de silicone telles que les cyclométhicones et les diméthicones, ou leurs mélanges.

Lorsque le composé biologiquement actif est un agent modulant la différenciation et/ou la prolifération, comme par exemple un rétinoide acide tel que l'acide 6-[7-(1-adamantyl)-6-methoxyethoxymethoxy-2-naphtyl] nicotinique, l'acide 6-[3-(1-adamantyl-4-hydroxyphenyl]-2-naphtoique, l'acide 6-(3,5,5,8,8-pentamethyl-5,6,7,8-tetrahydro-2-naphtylthio)nicotinique, l'acide 3-(3,5,5,8,8-pentamethyl-5,6,7,8-tetrahydro-2-naphtyl)phenylacrylique, ou l'acide 2-hydroxy-4-[7-(1-adamantyl)-6-methoxyethoxymethoxy-2-naphtyl] benzoique, les huiles sèches à moyennement sèches pouvant être utilisées dans les émulsions selon l'invention sont choisies préférentiellement dans le groupe formé par des hydrocarbures tel que le polyisobutène hydrogéné, l'isohexadécane vendue par la société ICI sous le nom de Arlamol HD, des huiles de silicone telles que les cyclométhicones et les diméthicones, ou leurs mélanges.

La phase aqueuse de l'émulsion selon l'invention peut comprendre de l'eau, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avère ou l'eau d'Aix les Bains.
Ladite phase aqueuse peut être présente à une teneur comprise entre 30 et 95 % en poids par rapport au poids total de la composition, de préférence comprise entre 60 et 80 % en poids.

Le pH de la composition selon l'invention est avantageusement compris entre 5 et 7, de préférence compris entre 5 et 6. Il sera ajusté à la valeur désirée par l'addition de bases ou d'acides usuels, minéraux ou organiques.

Les émulsions de l'invention peuvent contenir également un ou plusieurs agents mouillants dans des concentrations préférentielles allant de 0,1 à 10 % et plus préférentiellement allant de 2 à 2,5 %. Parmi les agents mouillants, on utilise préférentiellement, sans que cette liste soit limitative, des composés tels que les Poloxamers et plus particulièrement le Poloxamer 124 et/ou le Poloxamer 182, les esters de sorbitol oxyéthylénés tels que les Polysorbates et plus particulièrement le Polysorbate 60 et/ou le Polysorbate 80.

Les émulsions de l'invention peuvent contenir également un ou plusieurs agents propénétrants et/ou humectants dans des concentrations préférentielles allant de 1 à 20 % et plus préférentiellement allant de 2 à 6 %. Parmi les agents propénétrants et / ou humectants, on utilise préférentiellement, sans que cette liste soit limitative, des composés tels que le propylène glycol, la glycérine ou le sorbitol.

Les émulsions de l'invention peuvent contenir également un ou plusieurs agents gélifiants dans des concentrations préférentielles allant de 0,05 à 5 % et plus préférentiellement allant de 0,1 à 1 %. Parmi les agents gélifiants, on utilise préférentiellement, sans que cette liste soit limitative, des composés tels que les polymères carboxyvinyliques (Carbomer), les dérivés cellulosiques, comme par exemple l'hydroxypropylmethylcellulose, ou l'hydroxyethylcellulose; les gommes de xanthane, les gommes guar et semblables, les polyacrylamides tel que le mélange polyacrylamide / isoparaffine C13-14 / laureth-7 comme par exemple celui vendu par la société SEPPIC sous le nom de Sepigel 305, ou leurs mélanges.

L'émulsion peut comprendre en outre tout additif usuellement utilisé dans le domaine cosmétique ou pharmaceutique, tel que des séquestrants, des antioxydants, des filtres solaires, des conservateurs, des charges, des colorants, des parfums, des huiles essentielles, des actifs cosmétiques, des hydratants, des vitamines, des acides gras essentiels, des sphingolipides, des composés auto-bronzants tels que la DHA, , des agents apaisants et protecteurs de la peau tels que l'allantoïne. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.
Ces additifs peuvent être présents dans la composition à raison de 0 à 20% en poids par rapport au poids total de la composition.

On peut citer comme exemple d'agents séquestrants l'acide éthylènediamine tétracétique (EDTA), ainsi que ses dérivés ou ses sels, la dihydroxyethylglycine, l'acide citrique, l'acide tartrique, ou leurs mélanges.

On peut citer comme exemples de conservateurs le chlorure de benzalkonium, le phénoxyéthanol, l'alcool benzylique, la diazolidinylurée, les parabens, ou leurs mélanges.

Les exemples de formulations ci-dessous permettent d'illustrer les compositions selon l'invention. Les quantités des constituants sont exprimées en % en poids par rapport au poids total de la composition, sauf mention contraire.

### A- EXEMPLES DE FORMULATIONS

### Exemple 1

| Phase A | |
|---|---|
| Glycéryl stearate et PEG 100 stearate | 5,00 % |
| Polyisobutène hydrogéné | 11,00 % |
| propyl paraben | 0,10 % |
| acide stéarique | 2,00 % |

| Phase B | |
|---|---|
| Eau | qsp 100 % |
| Propylène glycol | 2 % |
| Edetate disodique | 0,10 % |
| Methyl paraben | 0,10 % |

| Phase C | |
|---|---|
| Nadifloxacine | 1,00 % |
| Poloxamer 124 | 2,00 % |
| Propylène glycol | 2,00 % |
| | |
| Copolymère d'acide acrylique et d'alkylmethacrylate | 0,20 % |
| cyclométhicone | 3,00 % |
| Hydroxyde de sodium à 10% | qs pH 5,5 |

### Mode opératoire:

On pèse les composés de la phase B et on les met sous agitation en chauffant. On incorpore ensuite le Copolymère d'acide acrylique et d'alkylmethacrylate.

On prépare par ailleurs la phase A par mélange et on chauffe la phase A au bain-marie à 75°C.
On verse la phase A dans la phase B, en maintenant la température à 75°C sous agitation. Puis on refroidit, et on incorpore la cyclométhicone et la phase active à 40°C. On ajuste le pH à 5,5 avec l'hydroxyde de sodium.
On obtient une émulsion stable, à un pH compatible avec la peau, confortable à l'étalement tout en évitant un effet collant, c'est à dire un véhicule adapté au traitement des pathologies visées.

### Exemple 2

| Phase A | |
|---|---|
| Isohexadecane | 5,00 % |
| Polyisobutène hydrogéné | 12,00 % |
| propyl paraben | 0,10 % |
| Sorbitan sesquiolate | 0,15 % |
| Ceteareth 20 | 0,25 *%* |

| Phase B | |
|---|---|
| Eau | qsp 100 % |
| Propylène glycol | 2,00 % |
| Edetate disodique | 0,10 % |
| Methyl paraben | 0,10 % |

| Phase C | |
|---|---|
| Nadifloxacine | 1,00 % |
| Poloxamer 124 | 2,00 % |
| Propylène glycol | 2,00 % |
| | |
| Copolymère d'acide acrylique et d'alkylmethacrylate | 0,35 % |
| carbomer | 0,10 % |
| Hydroxyde de sodium à 10% | qs pH 5,5 % |

### Mode opératoire :

Le mode opératoire utilisé est analogue au mode opératoire de l'exemple 1.
On obtient une émulsion stable, à un pH compatible avec la peau, confortable à l'étalement tout en évitant un effet collant, c'est à dire un véhicule adapté au traitement des pathologies visées.

### Exemple 3

| Phase A | |
|---|---|
| Diisopropyl adipate | 12,00 % |
| Ceteareth 20 | 0,25 % |

| phase B | |
|---|---|
| Eau | qsp 100 % |
| Propylène glycol | 2,00 % |
| Edetate disodique | 0,10 % |
| Chlorure de Benzalkonium | 0,05 % |

| Phase C | |
|---|---|
| Nadifloxacine | 1,00 % |
| Poloxamer 124 | 2,00 % |
| Propylène glycol | 2,00 % |
| | |
| Copolymère d'acide acrylique et d'alkylmethacrylate | 0,35 % |
| Carbomer 980 | 0,30 % |
| cyclométhicone | 5 % |
| Hydroxyde de sodium à 10% | qs pH 5,5 |

### Mode opératoire :

Le mode opératoire utilisé est analogue au mode opératoire de l'exemple 1.
On obtient une émulsion stable, à un pH compatible avec la peau, confortable à l'étalement tout en évitant un effet collant, c'est à dire un véhicule adapté au traitement des pathologies visées.

### Exemple 4

| Phase A | |
|---|---|
| Diisopropyl adipate | 15,00 % |
| Ceteareth 20 | 0,25 % |
| PPG 15 Stearyl ether vendu sous le nom de Arlamol E | 5 % |
| Propyl Paraben | 0,05 % |

| phase B | |
|---|---|
| Eau | qsp 100 % |
| Propylène glycol | 3,00 % |
| Edetate disodique | 0,10 % |
| Méthyl Paraben | 0,10 % |

| Phase C | |
|---|---|
| acide 3-(3,5,5,8,8-pentaméthyl-5,6,7,8-tetrahydro-2-naphtyl)phenyl | |
| acrylique | 1,00 % |
| Poloxamer 182 | 2,00 % |
| Propylène glycol | 2,00 % |
| | |
| Copolymère d'acide acrylique et d'alkylmethacrylate | 0,35 % |
| Carbomer 980 | 0,10 % |
| Chlorure de Benzalkonium | 0,10 % |
| Hydroxyde de sodium à 10% | qs pH 5,5 |

### Mode opératoire :

Le mode opératoire utilisé est analogue au mode opératoire de l'exemple 1.
On obtient une émulsion stable, à un pH compatible avec la peau, confortable à l'étalement tout en évitant un effet collant, c'est à dire un véhicule adapté au traitement des pathologies visées.

### Exemple 5

| Phase A | |
|---|---|
| isopropyl palmitate | 12,00 % |
| Ceteareth 20 | 0,40 % |
| cyclomethicone | 5 % |
| Propyl Paraben | 0,10 % |

| phase B | |
|---|---|
| Eau purifiée | qsp 100 % |
| Propylène glycol | 2,00 % |
| Edetate disodique | 0,10 % |
| Copolymère d'acide acrylique et d'alkylmethacrylate | 0,35 % |
| Carbomer 980 | 0,25 % |
| Phenoxyethanol | 1,00 % |

| Phase C | |
|---|---|
| Nadifloxacine | 1,00 % |
| Poloxamer 124 | 2,00 % |
| Propylène glycol | 2,00 % |
| | |
| Hydroxyde de sodium à 10% | qs pH 5,5 |

### Mode opératoire :

Le mode opératoire utilisé est similaire au mode opératoire de l'exemple 1.
On obtient une émulsion stable, à un pH compatible avec la peau, confortable à l'étalement tout en évitant un effet collant, c'est à dire un véhicule adapté au traitement des pathologies visées.

### B- RESULTATS DE STABILITE

Différentes émulsions de l'invention ont été testées en stabilité chimique. Les concentrations en composé biologiquement actif présentées dans le tableau ci-dessous ont été mesurées par HPLC :

Recouvrement : Pourcentage de Nadifloxacine retrouvée dans le produit par rapport à la quantité théorique introduite.

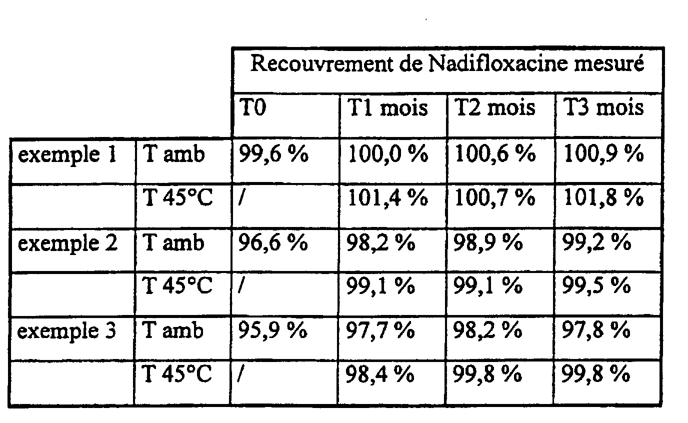

## Revendications

1. Composition cosmétique ou pharmaceutique du type émulsion huile-dans-eau comprenant une phase grasse dispersée dans une phase aqueuse, **caractérisée par le fait qu'**elle comprend :
A) au moins un composé biologiquement actif micronisé, non solubilisé, sous forme de particules, dont au moins 80% en nombre de particules et de préférence au moins 90% en nombre de particules ont un diamètre compris entre 1 à 10 µm et au moins 50 % en nombre de particules ont un diamètre inférieur à 5 µm, et
B) un système émulsionnant approprié.

2. Composition selon la revendication 1, **caractérisée par le fait que** le système émulsionnant approprié comprend au moins un copolymère constitué d'une fraction majoritaire de monomère acide carboxylique monooléfiniquement insaturé ayant pour formule : 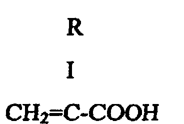 où R désigne hydrogène, halogène, hydroxyle, un groupe lactone, un groupe lactame, un groupe cyanogène (-C=N), un groupe alkyle monovalent, un groupe aryle, un groupe alkylaryle, un groupe aralkyle ou un groupe cycloaliphatique ou de son anhydride et d'une fraction minoritaire de monomère ester ayant pour structure : 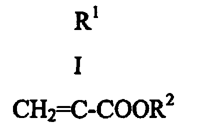 où R¹ est choisi dans le groupe formé par hydrogène, méthyle et éthyle et R² est un groupe allyle en C₈-C₃₀.

3. Composition selon la revendication 2, **caractérisée par le fait que** la quantité de monomère acide carboxylique ou de son anhydride varie de 80 à 98% en poids et que la quantité de monomère ester varie de 20 à 2% en poids ; les pourcentages en poids étant exprimés par rapport au poids total des deux monomères.

4. Composition selon la revendication 2, **caractérisée par le fait que** le monomère acide carboxylique est choisi parmi l'acide acrylique, l'acide méthacrylique ou leurs mélanges et que le monomère ester est choisi parmi ceux pour lesquels R¹ est hydrogène ou méthyle et R² est un groupe alkyle en C₁₀-C₂₂.

5. Composition selon l'une des revendications 2 à 4 **caractérisée en ce que** le copolymère utilisé est un copolymère acrylate/C₁₀-C₃₀-alkylacrylate.

6. Composition selon l'une quelconque des revendications 2 à 5, **caractérisée par le fait qu'**elle contient de 0,01 à 3% en poids de copolymère par rapport au poids total de la composition.

7. Composition selon la revendication 6 **caractérisée en ce qu'**elle contient de 0,05 à 2% et de préférence de 0,1 à 0,5% en poids de copolymère acrylate/C₁₀-C₃₀-alkylacrylate par rapport au poids total de la composition.

8. Composition selon l'une quelconque des revendications 1 à 7, **caractérisée par le fait qu'**elle contient au moins un émulsionnant tensioactif.

9. Composition selon la revendication 8 **caractérisée en ce que** l'émulsionnant tensioactif est choisi parmi le glyceryl PEG100 stéarate, les esters d'acide gras polyoxyéthylénés, l'alcool stéarylique polyoxyéthyléné (2) associé à l'alcool stéarylique polyéthyléné (21), ou leurs mélanges.

10. Composition selon l'une quelconque des revendications 1 à 9, **caractérisée par le fait qu'**elle contient au moins un cotensioactif.

11. Composition selon la revendication précédente **caractérisée en ce que** le cotensioactif est choisi parmi les esters de sorbitan, le sesquioleate de sorbitan, l'isostéarate de sorbitan, les éthers d'alcools gras ayant un haut HLB, les éthers d'alcools gras ayant un HLB bas, ou leurs mélanges.

12. Composition selon l'une quelconque des revendications 1 à 11, **caractérisée par le fait qu'**elle contient jusqu'à 15 % en poids et de préférence de 0,05 à 8% en poids et plus préférentiellement de 0,1 à 2% en poids de système émulsionnant approprié par rapport au poids total de la composition.

13. Composition selon l'une quelconque des revendications 1 à 12, **caractérisée en ce que** le composé biologiquement actif est choisi parmi tout composé biologiquement actif insoluble ou difficilement soluble dans l'eau ou un milieu hydrophile dans des conditions de pH compatible avec la peau, et susceptible d'être micronisé.

14. Composition selon l'une quelconque des revendications 1 à 13, **caractérisée en ce que** le composé biologiquement actif est choisi parmi les agents modulant la différenciation et/ou la prolifération et/ou la pigmentation cutanée, les antibactériens, les antiparasitaires, les antifongiques, les antibiotiques, les agents anti-inflammatoires stéroïdiens, les agents anti-inflammatoires non-stéroïdiens, les agents anesthésiques, les agents antiprurigineux, les agents antiviraux, les agents kératolytiques, les agents anti-radicaux libres, les antiséborrhéiques, les antipelliculaires, les antiacnéiques, les antimétabolites, les agents pour lutter contre la chute des cheveux et pour favoriser la repousse ou l'inverse, les antiseptiques ou leurs mélanges.

15. Composition selon l'une quelconque des revendications 1 à 14, **caractérisée par le fait qu'**elle contient de 0,0001 à 20% et de préférence de 0,025 à 15% en poids de composé biologiquement actif par rapport au poids total de la composition.

16. Composition selon l'une quelconque des revendications 1 à 15, **caractérisée par le fait qu'**elle contient de 5 à 50 % en poids de phase grasse par rapport au poids total de la composition et de préférence de 12 à 25 % en poids.

17. Composition selon la revendication 16, **caractérisée par le fait que** les corps gras de la phase grasse sont choisis parmi :
- les corps gras siliconés, comme les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA); les huiles siliconées volatiles, telles que les silicones volatiles cycliques ayant de 3 à 8 atomes de silicium et de préférence de 4 à 5, comme par exemple une cyclomethicone telle que la cyclotétradiméthylsiloxane, la cyclopentadiméthylsiloxane ou la cyclohexadiméthylsiloxane, les cyclocopolymères du type diméthylsiloxane/méthylalkylsiloxane, les silicones volatiles linéaires ayant de 2 à 9 atomes de silicium, comme par exemple de l'hexaméthyldisiloxane, de l'hexyl heptaméthyltrisiloxane ou de l'octyl heptaméthyltrisiloxane; les huiles de silicone phénylées, et/ou
- les corps gras non siliconés, comme les huiles usuelles, telles que l'isohexadécane, l'huile de paraffine, de vaseline, le perhydrosqualène, l'huile d'abricot, l'huile de germes de blé, d'amande douce, de calophyllum, de palme, de ricin, d'avocat, de jojoba, d'olive ou de germes de céréales; des esters d'acides gras ou d'alcools gras, telles que la diisopropyladipate, l'octyl dodécyl myristate ou les benzoates d'alkyle en C₁₂-C₁₅,; des acétylglycérides; des octanoates, décanoates ou ricinoléates d'alcools ou de polyalcools; des triglycérides d'acides gras; des glycérides; le polyisobutène hydrogéné, des huiles hydrogénées concrètes à 25°C; des lanolines; des esters gras concrets à 25°C, et/ou
- les autres corps gras, comme les acides gras tel que l'acide stéarique, les alcools gras tel que l'alcool stéarylique ou cétylique ou leurs dérivés, les cires ou leurs mélanges.

18. Composition selon l'une quelconque des revendications 1 à 17, **caractérisée par le fait qu'**elle contient de 30 à 95 % et de préférence de 60 à 80 % en poids de phase aqueuse par rapport au poids total de la composition.

19. Composition selon la revendication 18, **caractérisée par le fait que** la phase aqueuse comprend de l'eau choisie parmi une eau florale telle que l'eau de bleuet, ou une eau thermale ou minérale naturelle.

20. Composition selon l'une quelconque des revendications 1 à 19, **caractérisée par le fait qu'**elle contient en plus au moins un agent mouillant, de préférence choisi parmi les Poloxamers et plus particulièrement le Poloxamer 124 et/ou le Poloxamer 182, les esters de sorbitol oxyéthylénés tels que les Polysorbates et plus particulièrement le Polysorbate 60 et/ou le Polysorbate 80 ou leurs mélanges.

21. Composition selon la revendication 20, **caractérisée par le fait qu'**elle contient de 0,1 à 10 % et de préférence de 2 à 2,5 % en poids par rapport au poids total de la composition d'agent mouillant.

22. Composition selon l'une quelconque des revendications 1 à 21, **caractérisée par le fait qu'**elle contient en plus au moins un agent propénétrant et / ou humectant, de préférence choisi parmi le propylène glycol, la glycérine ou le sorbitol.

23. Composition selon la revendication 22, **caractérisée par le fait qu'**elle contient de 1 à 20 % et de préférence de 2 à 6 % en poids par rapport au poids total de la composition d'agent propénétrant et / ou humectant.

24. Composition selon l'une quelconque des revendications 1 à 23, **caractérisée par le fait qu'**elle contient en plus au moins un agent gélifiant, de préférence choisi parmi les polymères carboxyvinyliques, les dérivés cellulosiques, les gommes de xanthane, les gommes guar, les polyacrylamides, ou leurs mélanges.

25. Composition selon la revendication 24, **caractérisée par le fait qu'**elle contient de 0,05 à 5 % et de préférence de 0,1 à 1 % en poids par rapport au poids total de la composition d'agent gélifiant.

26. Composition selon l'une quelconque des revendications 1 à 25, **caractérisée par le fait qu'**elle contient des adjuvants, de préférence choisis parmi des séquestrants, des antioxydants, des filtres solaires, des conservateurs, des charges, des colorants, des parfums, des huiles essentielles, des actifs cosmétiques, des hydratants, des vitamines, des acides gras essentiels, des sphingolipides, des composés auto-bronzants, des agents apaisants et protecteurs de la peau, ou leurs mélanges.

27. Composition selon l'une quelconque des revendications 1 à 26, **caractérisée en ce qu'**elle est destinée, en application topique, au traitement ou au soin de la peau, et de préférence :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Damer, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation telles que la rosacée,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique,
7) pour prévenir ou guérir les stigmates de l'atrophie épidermique et/ou dermique induite par les corticostéroïdes locaux ou systémiques, ou toute autre forme d'atrophie cutanée,
8) pour traiter de manière préventive ou curative les troubles de la cicatrisation, pour prévenir ou pour réparer les vergetures, ou encore pour favoriser la cicatrisation,
9) pour lutter contre les troubles de la fonction sébacée tels que l'hyperséborrhée de l'acné ou la séborrhée simple,
10) dans le traitement préventif ou curatif des états cancéreux ou précancéreux,
11) dans le traitement d'affections inflammatoires telles que l'arthrite,
12) dans le traitement de toute affection d'origine virale au niveau cutané,
13) dans le traitement préventif ou curatif de l'alopécie,
14) dans le traitement d'affections dermatologiques à composante immunologique,
15) dans le traitement de désordres cutanés dus à une exposition aux rayonnements U.V.,
16) dans le traitement d'affections dermatologiques liées à une inflammation ou une infection des tissus environnants le follicule pileux, notamment dues à une colonisation ou infection microbienne notamment l'impétigo, la dermite seborrhéique, la folliculite, le sycosis barbae ou impliquant tout autre agent bactérien ou fongique,
17) dans les traitements cosmétiques destinés à accélérer ou favoriser la chute du poil.

28. Composition selon l'une quelconque des revendications 1 à 27, **caractérisée en ce qu'**elle est destinée à traiter, de manière préventive ou curative, l'acné.

29. Composition selon la revendication 28, **caractérisée par le fait qu'**elle contient de 0, 1 à 10% et de préférence de 0,5 à 2% en poids de composé biologiquement actif par rapport au poids total de la composition.

30. Composition selon l'une quelconque des revendications 28 à 29, **caractérisée par le fait que** le composé biologiquement actif est choisi dans le groupe formé par les agents modulant la prolifération et/ou la différentiation, les antibiotiques, les antibactériens, les antifongiques, les antiparasitaires, ou leurs mélanges.

31. Composition selon la revendication 30, **caractérisée par le fait que** le composé biologiquement actif est choisi parmi la nadifloxacine, l'acide 6-[7-(1-adamantyl)-6-methoxyethoxymethoxy-2-naphtyl] nicotinique, l'acide 6-[3-(1-adamantyl-4-hydroxyphenyl]-2-naphtoique, l'acide 6-(3,5,5,8,8-pentamethyl-5,6,7,8-tetrahydro-2-naphtylthio)nicotinique, l'acide 3-(3,5,5,8,8-pentamethyl-5,6,7,8-tetrahydro-2-naphtyl)phenylacrylique, l'acide 2-hydroxy-4-[7-(1-adamantyl)-6-methoxyethoxymethoxy-2-naphtyl] benzoique, ou leurs mélanges.

32. Composition selon la revendication 31, **caractérisée par le fait que** les corps gras de la phase grasse comprennent des huiles sèches à moyennement sèches.

33. Composition selon la revendication 32, **caractérisée par le fait que** les huiles sèches sont choisies parmi l'isohexadecane, le dioctyl cyclohexane, l'isopropyl palmitate, le polyisobutène hydrogéné, le diisopropyladipate, le dicaprylyl ether, l'isopropyl myristate, le dipropylène glycol diperlargonate, l'alkyl benzoate en C₁₂₋₁₅, le cetostearyl isononanoate, le cetostearyl ethylhexanoate, le squalene synthetique, l'huile d'olive, l'octyl palmitate, l'octyl dodecyl myristate, le caprylic / capric triglycérides,ou leurs mélanges.

34. Procédé de traitement cosmétique, **caractérisé par le fait qu'**on applique sur la peau ou le cuir chevelu la composition selon l'une quelconque des revendications 1 à 13 et 15 à 26, et en ce que la composition comprend un composé biologiquement actif choisi parmi les agents modulant la pigmentation cutanée, les agents kératolytiques, les antiséborrhéiques, les antipelliculaires, les agents pour lutter contre la chute des cheveux et pour favoriser la repousse ou l'inverse ou leurs mélanges

## Patentansprüche

1. Kosmetische oder pharmazeutische Zusammensetzung vom Typ einer Öl-in-Wasser-Emulsion, die eine in einer wässerigen Phase dispergierte Fettphase aufweist, **dadurch gekennzeichnet, dass** sie:
A) mindestens eine nicht solubilisierte, mikronisierte biologisch aktive Verbindung in Partikelform, wobei mindestens 80 % der Anzahl der Partikel und vorzugsweise mindestens 90 % der Anzahl der Partikel einen Durchmesser im Bereich von 1 bis 10 µm und mindestens 50 % der Anzahl der Partikel einen Durchmesser kleiner 5 µm aufweisen, und
B) ein geeignetes emulgierendes System enthält.

2. Zusammensetzung nach Anspruch 1, **dadurch gekennzeichnet, dass** das geeignete emulgierende System mindestens ein Copolymer aufweist, das zum überwiegenden Teil aus Monomeren einer einfach olefinisch ungesättigten Carbonsäure oder des Anhydrids dieser Säure, die der folgenden Formel entsprechen: 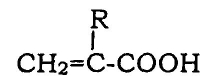 worin R Wasserstoff, Halogen, Hydroxy, eine Lactongruppe, eine Lactamgruppe, eine Cyanogruppe (-C=N), eine einwertige Alkylgruppe, eine Arylgruppe, eine Alkylarylgruppe, eine Aralkylgruppe oder eine cycloaliphatische Gruppe bedeutet, und zum kleineren Teil aus Estermonomeren der folgenden Struktur besteht: 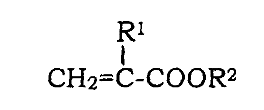 worin R¹ unter Wasserstoff, Methyl und Ethyl ausgewählt ist und worin R² eine Alkylgruppe mit 8 bis 30 Kohlenstoffatomen bedeutet.

3. Zusammensetzung nach Anspruch 2, **dadurch gekennzeichnet, dass** der Mengenanteil des Monomers der Carbonsäure oder ihres Anhydrids im Bereich von 80 bis 98 Gew.-% liegt und dass der Mengenanteil des Estermonomers im Bereich von 20 bis 2 Gew.-% liegt, wobei die Gewichtsprozentanteile bezogen auf das Gesamtgewicht der beiden Monomere ausgedrückt sind.

4. Zusammensetzung nach Anspruch 2, **dadurch gekennzeichnet, dass** das Carbonsäuremonomer unter Acrylsäure, Methacrylsäure oder den Gemischen dieser Verbindungen ausgewählt ist und dass das Estermonomer unter den Estermonomeren ausgewählt ist, worin R¹ Wasserstoff oder Methyl ist und R² eine C₁₀₋₂₂-Alkylgruppe bedeutet.

5. Zusammensetzung nach einem der Ansprüche 2 bis 4, **dadurch gekennzeichnet, dass** das verwendete Copolymer ein Acrylat/C₁₀₋₃₀-Alkylacrylat-Copolymer ist.

6. Zusammensetzung nach einem der Ansprüche 2 bis 5, **dadurch gekennzeichnet, dass** sie 0,01 bis 3 Gew.-% des Copolymers, bezogen auf das Gesamtgewicht der Zusammensetzung, enthält.

7. Zusammensetzung nach Anspruch 6, **dadurch gekennzeichnet, dass** sie 0,05 bis 2 Gew.-% und vorzugsweise 0,1 bis 0,5 Gew.-% eines Acrylat/C₁₀₋₃₀-Alkylacrylat-Copolymers, bezogen auf das Gesamtgewicht der Zusammensetzung, enthält.

8. Zusammensetzung nach einem der Ansprüche 1 bis 7, **dadurch gekennzeichnet, dass** sie mindestens einen grenzflächenaktiven Emulgator enthält.

9. Zusammensetzung nach Anspruch 8, **dadurch gekennzeichnet, dass** der grenzflächenaktive Emulgator unter Glyceryl-PEG-100 stearat, polyethoxylierten Fettsäureestern, polyethoxyliertem Stearylalkohol (2) in Kombination mit einem Stearylalkohol mit mehreren Ethyleneinheiten (21) oder den Gemischen dieser Verbindungen ausgewählt ist.

10. Zusammensetzung nach einem der Ansprüche 1 bis 9, **dadurch gekennzeichnet, dass** sie mindestens ein Cotensid enthält.

11. Zusammensetzung nach dem vorhergehenden Anspruch, **dadurch gekennzeichnet, dass** das Cotensid unter Sorbitanestern, Sorbitansesquioleat, Sorbitanisostearat, Ethern von Fettalkoholen mit hohem HLB-Wert, Ethern von Fettalkoholen mit niedrigem HLB-Wert oder den Gemischen dieser Verbindungen ausgewählt ist.

12. Zusammensetzung nach einem der Ansprüche 1 bis 11, **dadurch gekennzeichnet, dass** sie bis zu 15 Gew.-%, vorzugsweise 0,05 bis 8 Gew.-% und noch bevorzugter 0,1 bis 2 Gew.-% eines geeigneten emulgierenden Systems, bezogen auf das Gesamtgewicht der Zusammensetzung, enthält.

13. Zusammensetzung nach einem der Ansprüche 1 bis 12, **dadurch gekennzeichnet, dass** die biologisch aktive Verbindung unter beliebigen biologisch aktiven Verbindungen ausgewählt ist, die bei pH-Werten, die mit der Haut kompatibel sind, in Wasser oder in einem hydrophilen Medium unlöslich oder schwer löslich sind und die mikronisiert werden können.

14. Zusammensetzung nach einem der Ansprüche 1 bis 13, **dadurch gekennzeichnet, dass** die biologisch aktive Verbindung unter Wirkstoffen, die die Differenzierung und/oder Proliferation und/oder Pigmentierung der Haut modulieren, antibakteriellen Wirkstoffen, antiparasitären Wirkstoffen, Antimykotika, Antibiotika, steroidalen entzündungshemmenden Wirkstoffen, nichtsteroidalen entzündungshemmenden Wirkstoffen, Anästhetika, juckreizstillenden Wirkstoffen, antiviralen Wirkstoffen, Keratolytika, Radikalfängern für freie Radikale, Antiseborrhoeika, Antischuppenmitteln, Wirkstoffen gegen Akne, Antimetaboliten, Wirkstoffen zur Bekämpfung von Haarausfall und zur Förderung des Haarwuchses oder umgekehrt, Antiseptika oder deren Gemischen ausgewählt ist.

15. Zusammensetzung nach einem der Ansprüche 1 bis 14, **dadurch gekennzeichnet, dass** sie 0,0001 bis 20 Gew.-% und vorzugsweise 0,025 bis 15 Gew.-% der biologisch aktiven Verbindung, bezogen auf das Gesamtgewicht der Zusammensetzung, enthält.

16. Zusammensetzung nach einem der Ansprüche 1 bis 15, **dadurch gekennzeichnet, dass** sie 5 bis 50 Gew.-% und vorzugsweise 12 bis 25 Gew.-% Fettphase, bezogen auf das Gesamtgewicht der Zusammensetzung, enthält.

17. Zusammensetzung nach Anspruch 16, **dadurch gekennzeichnet, dass** die Fettsubstanzen der Fettphase ausgewählt sind unter:
- siliconhaltigen Fettsubstanzen, wie (C₁₋₂₀)-Polyalkylsiloxanen und insbesondere (C₁₋₂₀)-Polyalkylsiloxanen mit Trimethylsilyl-Endgruppen, vorzugsweise (C₁₋₂₀)-Polyalkylsiloxanen, deren Viskosität unter 0,06 m²/s liegt, von denen geradkettige Polydimethylsiloxane und Alkylmethylpolysiloxane, wie Cetyldimeticon (CTFA-Name), genannt werden können; flüchtigen Siliconölen, wie cyclischen flüchtigen Siliconen mit 3 bis 8 Siliciumatomen und vorzugsweise mit 4 bis 5 Siliciumatomen, beispielsweise einem Cyclometicon, wie Cyclotetradimethylsiloxan, Cyclopentadimethylsiloxan oder Cyclohexadimethylsiloxan, Cyclocopolymeren vom Typ Dimethylsiloxan/Methylalkylsiloxan, geradkettigen flüchtigen Siliconen mit 2 bis 9 Siliciumatomen, beispielsweise Hexamethyldisiloxan, Hexylheptamethyltrisiloxan oder Octylheptamethyltrisiloxan; und Phenylgruppen-haltigen Siliconölen und/oder
- nicht siliconhaltigen Fettsubstanzen, wie gebräuchlichen Ölen, wie Isohexadecan, Paraffinöl, Vaselineöl, Perhydrosqualen, Aprikosenöl, Weizenkeimöl, süßem Mandelöl, Calophyllumöl, Palmöl, Ricinusöl, Avocadoöl, Jojobaöl, Olivenöl oder Getreidekeimöl; Estern von Fettsäuren oder Fettalkoholen, wie Diisopropyladipat, Octyldodecylmyristat oder C₁₂₋₁₅-Alkylbenzoaten; Acetylglyceriden; Octanoaten, Decanoaten oder Ricinoleaten von Alkoholen oder Polyalkoholen; Triglyceriden von Fettsäuren; Glyceriden; hydriertem Polyisobuten, hydrierten Ölen, die bei 25 °C fest sind; Lanolinen und Fettestern, die bei 25 °C fest sind, und/oder
- weiteren Fettsubstanzen, wie Fettsäuren, wie Stearinsäure, Fettalkoholen, wie Stearylalkohol oder Cetylalkohol, oder deren Derivaten, Wachsen oder den Gemischen dieser Verbindungen.

18. Zusammensetzung nach einem der Ansprüche 1 bis 17, **dadurch gekennzeichnet, dass** sie 30 bis 95 Gew.-% und vorzugsweise 60 bis 80 Gew.-% wässerige Phase, bezogen auf das Gesamtgewicht der Zusammensetzung, enthält.

19. Zusammensetzung nach Anspruch 18, **dadurch gekennzeichnet, dass** die wässerige Phase Wasser enthält, das unter einem Blütenwasser, wie Kornblumenwasser, oder einem natürlichen Thermalwasser oder Mineralwasser ausgewählt ist.

20. Zusammensetzung nach einem der Ansprüche 1 bis 19, **dadurch gekennzeichnet, dass** sie ferner mindestens ein Netzmittel enthält, das vorzugsweise unter Poloxameren und insbesondere Poloxamer 124 und/oder Poloxamer 182, ethoxylierten Sorbitanestern, wie Polysorbaten und insbesondere Polysorbat 60 und/oder Polysorbat 80, oder deren Gemischen ausgewählt ist.

21. Zusammensetzung nach Anspruch 20, **dadurch gekennzeichnet, dass** sie 0,1 bis 10 Gew.-% und vorzugsweise 2 bis 2,5 Gew.-% des Netzmittels, bezogen auf das Gesamtgewicht der Zusammensetzung, enthält.

22. Zusammensetzung nach einem der Ansprüche 1 bis 21, **dadurch gekennzeichnet, dass** sie ferner mindestens einen penetrationsfördernden Wirkstoff und/oder ein Feuchthaltemittel enthält, die vorzugsweise unter Propylenglykol, Glycerin oder Sorbit ausgewählt sind.

23. Zusammensetzung nach Anspruch 22, **dadurch gekennzeichnet, dass** sie 1 bis 20 Gew.-% und vorzugsweise 2 bis 6 Gew.-% des penetrationsfördernden Wirkstoffs und/oder Feuchthaltemittels, bezogen auf das Gesamtgewicht der Zusammensetzung, enthält.

24. Zusammensetzung nach einem der Ansprüche 1 bis 23, **dadurch gekennzeichnet, dass** sie außerdem mindestens einen Gelbildner enthält, der unter Carboxyvinylpolymeren, Cellulosederivaten, Xanthangummen, Guargummen, Polyacrylamiden oder den Gemischen dieser Verbindungen ausgewählt ist.

25. Zusammensetzung nach Anspruch 24, **dadurch gekennzeichnet, dass** sie 0,05 bis 5 Gew.-% und vorzugsweise 0,1 bis 1 Gew.-% des Gelbildners, bezogen auf das Gesamtgewicht der Zusammensetzung, enthält.

26. Zusammensetzung nach einem der Ansprüche 1 bis 25, **dadurch gekennzeichnet, dass** sie Zusatzstoffe enthält, die vorzugsweise unter Maskierungsmitteln, Antioxidantien, Sonnenschutzfiltern, Konservierungsstoffen, Füllstoffen, Farbstoffen, Parfums, etherischen Ölen, kosmetischen Wirkstoffen, Hydratisierungsmitteln, Vitaminen, essentiellen Fettsäuren, Sphingolipiden, selbstbräunenden Verbindungen, beruhigenden Wirkstoffen, Wirkstoffen zum Schutz der Haut oder den Gemischen dieser Verbindungen ausgewählt sind.

27. Zusammensetzung nach einem der Ansprüche 1 bis 26, **dadurch gekennzeichnet, dass** sie zur topischen Anwendung für die Behandlung oder die Pflege der Haut vorgesehen ist und vorzugsweise:
1) zur Behandlung von Hautkrankheiten, die mit einer Verhornungsstörung verbunden sind und die Differenzierung und Proliferation betreffen, insbesondere zur Behandlung von Acne vulgaris, Acne comedonica, polymorpher Akne, Rosacea, nodulocystischer Akne, Acne conglobata, Acne senilis sowie sekundären Akneformen, wie Acne solaris, medikamentöser Akne oder Acne professionalis;
2) zur Behandlung weiterer Arten von Verhornungsstörungen, insbesondere Ichthyosen, ichthyosiformen Zuständen, der Darier-Krankheit, Palmoplantarkeratosen, Leukoplakie und leukoplakiformen Zuständen sowie Lichen der Haut oder der Schleimhäute (bukkal);
3) zur Behandlung weiterer Hautkrankheiten, die mit einer Verhornungsstörung mit einer entzündlichen und/oder immunallergischen Komponente verbunden sind, und insbesondere sämtlicher Formen der Psoriasis der Haut, der Schleimhäute oder der Nägel, und sogar zur Behandlung von arthropathischer Psoriasis oder auch der Atopie der Haut, wie Ekzemen, Atopie der Atemwege oder Hypertrophie des Zahnfleischs; die Verbindungen können ferner bei verschiedenen entzündlichen Erkrankungen verwendet werden, bei denen keine Verhornungsstörung vorliegt, wie Rosacea;
4) zur Behandlung sämtlicher Proliferationen der Dermis oder Epidermis, die gutartig oder bösartig und die gegebenenfalls viralen Ursprungs sein können, wie beispielsweise von Verrucae vulgares, Verrucae planae, Epidermodysplasia verruciformis, oralen oder floriden Papillomatosen sowie Proliferationen, die durch ultraviolette Strahlung hervorgerufen werden können, insbesondere im Fall von Basaliomen und Plattenepithelkarzinomen;
5) zur Behandlung weiterer Hautstörungen, wie bullöser Dermatosen und Kollagenosen;
6) zur Behebung oder zur Bekämpfung der Hautalterung, die lichtinduziert oder altersbedingt sein kann, oder zur Verminderung der Pigmentierung und von Lichtkeratosen oder beliebigen Krankheiten, die mit der altersbedingten oder aktinischen Alterung verbunden sind;
7) zur Vorbeugung oder Bekämpfung von Zeichen der durch lokale oder systemische Corticosteroide ausgelösten epidermalen und/oder dermalen Atrophie oder beliebiger weiterer Formen der Atrophie der Haut;
8) zur Vorbeugung oder Bekämpfung von Störungen der Wundheilung oder zur Vorbeugung oder Behebung von atrophischen Hautstreifen, oder auch zur Förderung der Wundheilung;
9) zur Bekämpfung von Störungen der Talgdrüsenfunktion, wie der übermäßigen Talgproduktion der Akne oder der einfachen Seborrhoe;
10) bei der Vorbeugung oder Behandlung von kanzerösen oder präkanzerösen Zuständen;
11) bei der Behandlung von entzündlichen Erkrankungen, wie Arthritis;
12) bei der Behandlung beliebiger Hauterkrankungen viralen Ursprungs;
13) bei der Vorbeugung oder Behandlung von Alopezie;
14) bei der Behandlung von Hautkrankheiten mit immunologischer Komponente;
15) bei der Behandlung von Hautstörungen, die von der Bestrahlung mit UV-Strahlung herrühren;
16) bei der Behandlung von Hautkrankheiten, die mit einer Entzündung oder einer Infektion der die Haarfollikel umgebenden Gewebe verbunden sind und die insbesondere durch bakterielle Kolonisation oder Infektion verursacht werden, insbesondere Impetigo, seborrhoischer Dermitis, Follikulitis, Sycosis barbae oder Hauterkrankungen, an denen weitere Bakterien oder Pilze beteiligt sind;
17) bei kosmetischen Behandlungen zur Beschleunigung oder Förderdung des Haarausfalls.

28. Zusammensetzung nach einem der Ansprüche 1 bis 27, **dadurch gekennzeichnet, dass** sie zur Vorbeugung oder zur Bekämpfung von Akne bestimmt ist.

29. Zusammensetzung nach Anspruch 28, **dadurch gekennzeichnet, dass** sie 0,1 bis 10 Gew.-% und vorzugsweise 0,5 bis 2 Gew.-% der biologisch aktiven Verbindung, bezogen auf das Gesamtgewicht der Zusammensetzung, enthält.

30. Zusammensetzung nach einem der Ansprüche 28 bis 29, **dadurch gekennzeichnet, dass** die biologisch aktive Verbindung unter Wirkstoffen, die die Proliferation und/oder Differenzierung modulieren, Antibiotika, antibakteriellen Wirkstoffen, Antimykotika, antiparasitären Wirkstoffen oder den Gemischen dieser Verbindungen ausgewählt ist.

31. Zusammensetzung nach Anspruch 30, **dadurch gekennzeichnet, dass** die biologisch aktive Verbindung unter Nadifloxacin, 6-[7-(1-Adamantyl)-6-methoxyethoxymethoxy-2-naphthyl]-nicotinsäure, 6-[3-(1-Adamantyl)-4-hydroxyphenyl]-2-naphthoesäure, 6-(3,5,5,8,8-Pentamethyl-5,6,7,8-tetrahydro-2-naphthylthio)-nicotinsäure, 3-(3,5, 5,8,8-Pentamethyl-5,6,7,8-tetrahydro-2-naphthyl)-phenylacrylsäure, 2-Hydroxy-4-[7-(1-adamantyl)-6-methoxyethoxymethoxy-2-naphthyl]-benzoesäure oder den Gemischen dieser Verbindungen ausgewählt ist.

32. Zusammensetzung nach Anspruch 31, **dadurch gekennzeichnet, dass** die Fettsubstanzen der Fettphase trockene bis mitteltrockene Öle umfassen.

33. Zusammensetzung nach Anspruch 32, **dadurch gekennzeichnet, dass** die trockenen Öle unter Isohexadecan, Dioctylcyclohexan, Isopropylpalmitat, hydriertem Polyisobuten, Diisopropyladipat, Dicaprylylether, Isopropylmyristat, Dipropylenglykoldipelargonat, C₁₂₋₁₅-Alkylbenzoat, Cetostearylisononanoat, Cetostearylethylhexanoat, synthetischem Squalen, Olivenöl, Octylpalmitat, Octyldodecylmyristat, Capryl-/Caprinsäure-Triglyceriden oder den Gemischen dieser Verbindungen ausgewählt sind.

34. Verfahren zur kosmetischen Behandlung, **dadurch gekennzeichnet, dass** die Zusammensetzung nach einem der Ansprüche 1 bis 13 und 15 bis 26 auf die Haut oder die Kopfhaut aufgebracht wird, und dadurch, dass die Zusammensetzung eine biologisch aktive Verbindungen enthält, die unter Wirkstoffen, die die Pigmentierung der Haut modulieren, Keratolytika, Antiseborrhoeika, Antischuppenmitteln, Wirkstoffen zur Bekämpfung von Haarausfall und zur Förderung des Haarwuchses oder umgekehrt oder den Gemischen dieser Verbindungen ausgewählt ist.

## Claims

1. Cosmetic or pharmaceutical composition of oil-in-water emulsion type comprising a fatty phase dispersed in an aqueous phase, **characterized in that** it comprises:
A) at least one non-solubilized, micronized, biologically active compound in particle form, in which at least 80%, in numerical terms, of the particles and preferably at least 90%, in numerical terms, of the particles have a diameter of between 1 and 10 µm and at least 50%, in numerical terms, of the particles have a diameter of less than 5 µm, and
B) a suitable emulsifying system.

2. Composition according to Claim 1, **characterized in that** the suitable emulsifying system comprises at least one copolymer consisting of a major fraction of monoolefinically unsaturated carboxylic acid monomer having the formula: 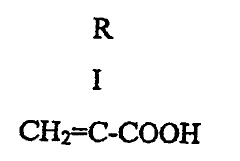 in which R denotes hydrogen, halogen, hydroxyl, a lactone group, a lactam group, a cyanogen (-C=N) group, a monovalent alkyl group, an aryl group, an alkylaryl group, an aralkyl group or a cycloaliphatic group, or its anhydride and a minor fraction of ester monomer having the structure: 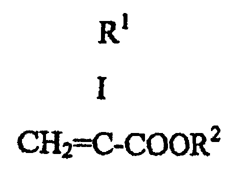 in which R¹ is chosen from the group formed by hydrogen, methyl and ethyl and R² is a C₈-C₃₀ alkyl group.

3. Composition according to Claim 2, **characterized in that** the amount of carboxylic acid monomer or its anhydride ranges from 80 to 98% by weight and **in that** the amount of ester monomer ranges from 20 to 2% by weight; the weight percentages being expressed relative to the total weight of the two monomers.

4. Composition according to Claim 2, **characterized in that** the carboxylic acid monomer is chosen from acrylic acid and methacrylic acid or mixtures thereof and **in that** the ester monomer is chosen from those for which R¹ is hydrogen or methyl and R² is a C₁₀-C₂₂ alkyl group.

5. Composition according to one of Claims 2 to 4, **characterized in that** the copolymer used is an acrylate/C₁₀-C₃₀-alkylacrylate copolymer.

6. Composition according to any one of Claims 2 to 5, **characterized in that** it contains from 0.01 to 3% by weight of copolymer relative to the total weight of the composition.

7. Composition according to Claim 6, **characterized in that** it contains from 0.05 to 2% and preferably from 0.1 to 0.5% by weight of acrylate/C₁₀-C₃₀-alkylacrylate copolymer relative to the total weight of the composition.

8. Composition according to any one of Claims 1 to 7, **characterized in that** it contains at least one surfactant emulsifier.

9. Composition according to Claim 8, **characterized in that** the surfactant emulsifier is chosen from glyceryl PEG-100 stearate, polyoxyethylenated fatty acid esters, polyoxyethylenated stearyl alcohol (2) combined with polyethylenated stearyl alcohol (21), or mixtures thereof.

10. Composition according to any one of Claims 1 to 9, **characterized in that** it contains at least one co-surfactant.

11. Composition according to the preceding claim, **characterized in that** the co-surfactant is chosen from sorbitan esters, sorbitan sesquioleate, sorbitan isostearate, fatty alcohol ethers with a high HLB value and fatty alcohol ethers with a low HLB value, or mixtures thereof.

12. Composition according to any one of Claims 1 to 11, **characterized in that** it contains up to 15% by weight and preferably from 0.05 to 8% by weight and more preferably from 0.1 to 2% by weight of suitable emulsifying system relative to the total weight of the composition.

13. Composition according to any one of Claims 1 to 12, **characterized in that** the biologically active compound is chosen from any biologically active compound which is insoluble or difficult to dissolve in water or a hydrophilic medium under pH conditions which are compatible with the skin, and which can be micronized.

14. Composition according to any one of Claims 1 to 13, **characterized in that** the biologically active compound is chosen from agents which modulate skin differentiation and/or proliferation and/or pigmentation, antibacterial agents, antiparasitic agents, antifungal agents, antibiotics, steroidal anti-inflammatory agents, non-steroidal anti-inflammatory agents, anaesthetics, anti-pruriginous agents, antiviral agents, keratolytic agents, free-radical scavengers, antiseborrhoeic agents, antidandruff agents, anti-acne agents, antimetabolites, agents for combating hair loss and for promoting hair growth or vice versa, and antiseptics, or mixtures thereof.

15. Composition according to any one of Claims 1 to 14, **characterized in that** it contains from 0.0001 to 20% and preferably from 0.025 to 15% by weight of biologically active compound relative to the total weight of the composition.

16. Composition according to any one of Claims 1 to 15, **characterized in that** it contains from 5 to 50% by weight of fatty phase relative to the total weight of the composition and preferably from 12 to 25% by weight.

17. Composition according to Claim 16, **characterized in that** the fatty substances in the fatty phase are chosen from:
- silicone fatty substances, such as poly(C₁-C₂₀)alkylsiloxanes and in particular those containing trimethylsilyl end groups, preferably those whose viscosity is less than 0.06 m²/s, among which mention may be made of linear polydimethylsiloxanes and alkylmethylpolysiloxanes such as cetyldimethicone (CTFA name); volatile silicone oils, such as cyclic volatile silicones containing from 3 to 8 and preferably from 4 to 5 silicon atoms, such as, for example, a cyclomethicone such as cyclotetradimethylsiloxane, cyclopentadimethylsiloxane or cyclohexadimethylsiloxane, cyclocopolymers such as dimethylsiloxane/methylalkylsiloxane, linear volatile silicones containing from 2 to 9 silicon atoms, such as, for example, hexamethyldisiloxane, hexyl heptamethyltrisiloxane or octyl heptamethyltrisiloxane; phenylsilicone oils, and/or
- non-silicone fatty substances, for instance the usual oils, such as isohexadecane, liquid paraffin, liquid petroleum jelly, perhydrosqualene, apricot oil, wheat germ oil, sweet almond oil, beauty-leaf oil, palm oil, castor oil, avocado·oil, jojoba oil, olive oil or cereal germ oil; esters of fatty acids or of fatty alcohols, such as diisopropyl adipate, octyldodecyl myristate or (C₁₂-C₁₅)alkyl benzoates; acetyl glycerides; alcohol or polyalcohol octanoates, decanoates or ricinoleates; fatty acid triglycerides; glycerides; hydrogenated polyisobutene, hydrogenated oils that are solid at 25°C; lanolins; fatty esters that are solid at 25°C, and/or
- other fatty substances which may be mentioned are fatty acids such as stearic acid, fatty alcohols such as stearyl alcohol or cetyl alcohol or derivatives thereof, and waxes, or mixtures thereof.

18. Composition according to any one of Claims 1 to 17, **characterized in that** it contains from 30 to 95% and preferably from 60 to 80% by weight of aqueous phase relative to the total weight of the composition.

19. Composition according to Claim 18, **characterized in that** the aqueous phase comprises water chosen from a floral water such as cornflower water, or a thermal spring water or natural mineral water.

20. Composition according to any one of Claims 1 to 19, **characterized in that** it also contains at least one wetting agent preferably chosen from Poloxamers and more particularly Poloxamer 124 and/or Poloxamer 182, oxyethylenated sorbitol esters such as Polysorbates and more particularly Polysorbate 60 and/or Polysorbate 80, or mixtures thereof.

21. Composition according to Claim 20, **characterized in that** it contains from 0.1 to 10% and preferably from 2 to 2.5% by weight, relative to the total weight of the composition, of wetting agent.

22. Composition according to any one of Claims 1 to 21, **characterized in that** it also contains at least one pro-penetrating and/or wetting agent, preferably chosen from propylene glycol, glycerol or sorbitol.

23. Composition according to Claim 22, **characterized in that** it contains from 1 to 20% and preferably from 2 to 6% by weight, relative to the total weight of the composition, of pro-penetrating and/or wetting agent.

24. Composition according to any one of Claims 1 to 23, **characterized in that** it also contains at least one gelling agent preferably chosen from carboxyvinyl polymers, cellulose derivatives, xanthan gums, guar gums and polyacrylamides, or mixtures thereof.

25. Composition according to Claim 24, **characterized in that** it contains from 0.05 to 5% and preferably from 0.1 to 1% by weight, relative to the total weight of the composition, of gelling agent.

26. Composition according to any one of Claims 1 to 25, -**characterized in that** it contains adjuvants preferably chosen from sequestering agents, antioxidants, sunscreens, preserving agents, fillers, dyes, fragrances, essential oils, cosmetic active agents, moisturizers, vitamins, essential fatty acids, sphingolipids, self-tanning compounds and agents for soothing and protecting the skin, or mixtures thereof.

27. Composition according to any one of Claims 1 to 26, **characterized in that** it is intended, in topical application, to treat or care for the skin, and preferably:
1) for treating dermatological complaints associated with a keratinization disorder which has a bearing on differentiation and proliferation, in particular for treating common acne, comedones, polymorphonuclear leukocytes, rosacea, nodulocystic acne, acne conglobata, senile acne and secondary acne such as solar, medication-related or occupational acne,
2) for treating other types of keratinization disorders, in particular ichtyosis, ichtyosiform states, Darier's disease, palmoplantar keratoderma, leukoplasia and leukoplasiform states, and cutaneous or mucous (buccal) lichen,
3) for treating other dermatological complaints associated with a keratinization disorder with an inflammatory and/or immunoallergenic component and, in particular, all forms of psoriasis, whether cutaneous, mucous or ungual psoriasis, and even psoriatic rheumatism, or alternatively cutaneous atopy, such as eczema or respiratory atopy or alternatively gingival hypertrophy; the compounds can also be used in certain inflammatory complaints which have no keratinization disorder, such as rosacea,
4) for treating all dermal or epidermal proliferations, whether benign or malignant and whether of viral or non-viral origin, such as common warts, flat warts and verruciform epidermodysplasia, oral or florid papillomatoses and proliferations which may be induced by ultraviolet radiation, in particular in the case of basocellular and spinocellular epithelioma,
5) for treating other dermatological disorders such as bullosis and collagen diseases,
6) for repairing or combating ageing of the skin, whether this is light-induced or chronological ageing, or for reducing pigmentations and actinic keratosis, or any pathologies associated with chronological or actinic ageing,
7) for preventing or curing the stigmata of epidermal and/or dermal atrophy induced by local or systemic corticosteroids, or any other form of cutaneous atrophy,
8) for the preventive or curative treatment of cicatrization disorders, for preventing or repairing stretch marks or for promoting cicatrization,
9) for combating disorders of sebaceous functioning, such as acneic hyperseborrhoea or simple seborrhoea,
10) in the preventive or curative treatment of cancerous or precancerous states,
11) in the treatment of inflammatory complaints such as arthritis,
12) in the treatment of any skin complaint of viral origin,
13) in the preventive or curative treatment of alopecia,
14) in the treatment of dermatological complaints with an immunological component,
15) in the treatment of skin disorders due to exposure to UV radiation,
16) in the treatment of dermatological complaints associated with inflammation or infection of the tissues surrounding the hair follicles, in particular due to microbial colonization or infection, in particular impetigo, seborrhoeic dermatitis, folliculitis or sycosis barbae or involving any other bacterial or fungal agent,
17) in cosmetic treatments intended to accelerate or promote hair loss.

28. Composition according to any one of Claims 1 to 27, **characterized in that** it is intended for the preventive or curative treatment of acne.

29. Composition according to Claim 28, **characterized in that** it contains from 0.1 to 10% and preferably from 0.5 to 2% by weight of biologically active compound relative to the total weight of the composition.

30. Composition according to either of Claims 28 and 29, **characterized in that** the biologically active compound is chosen from the group formed by agents which modulate proliferation and/or differentiation, antibiotics, antibacterial agents, antifungal agents and antiparasitic agents, or mixtures thereof.

31. Composition according to Claim 30, **characterized in that** the biologically active compound is chosen from nadifloxacin, 6-[7- (1-adamantyl) -6-methoxy-ethoxymethoxy-2-naphthyl]nicotinic acid, 6-[3-(1-adamantyl-4-hydroxyphenyl]-2-naphthoic acid, 6-(3,5,5,8,8-pentamethyl-5,6,7,8-tetrahydro-2-naphthylthio)nicotinic acid, 3-(3,5,5,8,8-pentamethyl-5,6,7,8-tetrahydro-2-naphthyl)phenylacrylic acid or 2-hydroxy-4-[7-(1-adamantyl)-6-methoxy-ethoxymethoxy-2-naphthyl]benzoic acid, or mixtures thereof.

32. Composition according to Claim 31, **characterized in that** the fatty substances in the fatty phase comprise dry to moderately dry oils.

33. Composition according to Claim 32, **characterized in that** the dry oils are chosen from isohexadecane, dioctylcyclohexane, isopropyl palmitate, hydrogenated polyisobutene, diisopropyl adipate, dicaprylyl ether, isopropyl myristate, dipropylene glycol dipelargonate, C₁₂₋₁₅ alkyl benzoate, cetostearyl isononanoate, cetostearyl ethylhexanoate, synthetic squalene, olive oil, octyl palmitate, octyldodecyl myristate, caprylic/capric triglycerides, or mixtures thereof.

34. Cosmetic treatment process, **characterized in that** the composition according to any one of Claims 1 to 13 and 15 to 26 is applied to the skin or the scalp and **in that** the composition comprises a biologically active compound chosen from the agents which modulate skin pigmentation, keratolytic agents, antiseborrhoeic agents, antidandruff agents and agents for combating hair loss and for promoting hair growth or vice versa, or mixtures thereof.
